(19) Europäisches Patentamt
European Patent Office
Office européen des brevets

(11) EP 1 297 819 A2

(12) **DEMANDE DE BREVET EUROPEEN**

(43) Date de publication:
**02.04.2003 Bulletin 2003/14**

(51) Int Cl.⁷: **A61K 7/13**

(21) Numéro de dépôt: **02292149.8**

(22) Date de dépôt: **30.08.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **26.09.2001 FR 0112374**

(71) Demandeur: **L'OREAL**
**75008 Paris (FR)**

(72) Inventeurs:
- **Vidal, Laurent**
**75013 Paris (FR)**
- **Fadli, Aziz**
**77500 Chelles (FR)**

(74) Mandataire: **Lhoste, Catherine**
**L'OREAL-DPI**
**6 rue Bertrand Sincholle**
**92585 Clichy Cédex (FR)**

(54) **Composition pour la teinture des fibres kératiniques comprenant un colorant azoique cationique particulier**

(57) L'invention a pour objet une nouvelle composition tinctoriale pour la teinture des fibres kératiniques humaines, et en particulier des cheveux, comprenant un colorant monoazoïque monocationique de formule (I)

$$W_1—W_2—N{=}N—W_3$$

ainsi que le procédé de teinture la mettant en oeuvre et les composés nouveaux de formule (I).

EP 1 297 819 A2

## Description

L'invention a pour objet une composition tinctoriale pour la teinture des fibres kératiniques humaines et plus particulièrement des cheveux, comprenant un colorant monoazoïque monocationique particulier, ainsi que les procédés de teinture des fibres kératiniques humaines mettant en oeuvre une telle composition. L'invention a aussi pour objet des composés monoazoïques monocationiques nouveaux.

Il est connu de teindre les fibres kératiniques humaines et en particulier les cheveux avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur le fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques humaines par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres.

Il est connu par exemple d'utiliser des colorants directs nitrés benzèniques, anthraquinoniques, nitropyridiniques, des colorants azoïques, xanthéniques, acridiniques aziniques ou des colorants triarylméthaniques.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Il est connu d'utiliser des colorants directs en combinaison avec des agents oxydants. Cependant, les colorants directs sont généralement sensibles à l'action des agents oxydants tels que l'eau oxygénée, et les agents réducteurs tels que le bisulfite de sodium, ce qui les rend généralement difficilement utilisables dans les compositions de teinture directe éclaircissante à base d'eau oxygénée et à base d'un agent alcalinisant ou dans des compositions de teinture d'oxydation en association avec des précurseurs du type bases d'oxydation ou coupleurs.

Par exemple, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.

On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

Plus récemment, la demande de brevet FR 2 741 798 a décrit des compositions tinctoriales contenant des colorants directs comportant au moins un atome d'azote quaternisé du type azoïque ou azométhine, lesdites compositions étant à mélanger extemporanément à pH basique à une composition oxydante. Ces compositions permettent

d'obtenir des colorations avec des reflets homogènes, tenaces et brillants. Cependant, elles ne permettent pas de teindre les fibres kératiniques avec autant de puissance qu'avec des compositions de coloration d'oxydation.

Il existe donc un réel besoin de rechercher des colorants directs chromatiques qui permettent de teindre les fibres kératiniques humaines aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation à base de précurseurs de colorants d'oxydation les contenant. Il existe aussi un réel besoin de rechercher des colorants directs qui permettent de teindre les fibres kératiniques humaines pour obtenir une gamme très large de couleurs, en particulier très chromatiques, sans oublier les nuances dites « fondamentale » comme les noirs et les marrons.

Ces buts sont atteints avec la présente invention qui a pour objet une composition pour la teinture des fibres kératiniques humaines et plus particulièrement des cheveux, comprenant dans un milieu cosmétiquement acceptable, au moins un colorant monoazoïque monocationique de formule (I) suivante :

$$W_1—W_2—N{=}N—W_3$$

Formule (I)

dans laquelle

$W_1$ représente un hétérocycle à 5, 6,7 ou 8 chaînons de formule (II) suivante

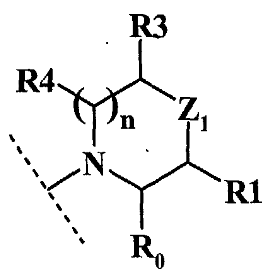

formule (II)

$W_2$ représente un groupe divalent aromatique carboné, pyridinique ou pyridazinique de formule (III) suivante

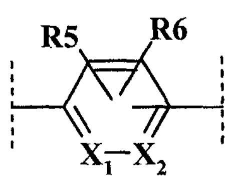

formule (III)

$W_3$ représente un radical hétéroaromatique cationique représenté par la formule (IV) suivante :

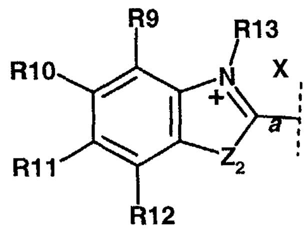

(IV)

Formules (II), (III), (IV) dans lesquelles :

- n = 0, 1, 2 ou 3, étant entendu que lorsque n est supérieur ou égal à 2, alors les radicaux $R_4$ peuvent être identiques ou différents,
- $X_1$ représente un atome d'azote ou un radical $CR_7$,
- $X_2$ représente un atome d'azote ou un radical $CR_8$,
- $Z_1$ représente un radical $CHR_2$ , un atome d'oxygène, de soufre ou un radical $NR_{14}$,
- $Z_2$ représente un atome d'oxygène, de soufre ou un radical $NR_{15}$
- $R_{0,}$ $R_1$, $R_2$, $R_3$, $R_4$, $R_5$, $R_6$, $R_7$, $R_8$, $R_9$, $R_{10}$, $R_{11}$ et $R_{12}$ représentent, identiques ou différents, un atome d'hydrogène, une chaîne hydrocarbonée en $C_1$-$C_{10}$ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement $SO_2$, et dont les atomes de carbone peuvent être, indépendamment les uns des autres, substitués par un ou plusieurs atomes d'halogène ; $R_{0,}$ $R_1$, $R_2$, $R_3$, $R_4$, $R_5$, $R_6$, $R_7$, $R_8$, $R_9$, $R_{11}$ et $R_{12}$ ne comportant pas de liaison peroxyde, ni de radicaux diazo ou nitroso,
- $R_{14}$ représente un atome d'hydrogène, une chaîne hydrocarbonée en $C_1$-$C_{10}$ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement $SO_2$, et dont les atomes de carbone peuvent être, indépendamment les uns des autres, substitués par un ou plusieurs atomes d'halogène, $R_{14}$ ne comportant pas de liaison peroxyde, ni de radicaux diazo ou nitroso ; étant entendu que lesdits atomes d'oxygène, d'azote et de soufre ne sont pas reliés directement à l'atome d'azote porteur du radical $R_{14}$,
- $R_5$ avec $R_6$ peuvent former un cycle aromatique carboné, tel qu'un phényle,
- $R_{13}$ et $R_{15}$ représentent, identiques ou différents, un radical alkyle en $C_1$-$C_8$, éventuellement substitué par un ou plusieurs radicaux choisis dans le groupe consitué par un hydroxy, un alcoxy en $C_1$-$C_2$, un radical (poly)hydroxyalcoxy en $C_2$-$C_4$, un amino, un (di)-alkylamino en $C_1$-$C_2$, un carboxyle, un sulfonique, un phényl éventuellement substitué ;
- la liaison **a** du cycle cationique de la formule (IV) est reliée au groupement azoïque de la formule (I);
- X est un anion organique ou minéral.

Selon l'invention, lorsqu'il est indiqué qu'un ou plusieurs des atomes de carbone de la chaîne hydrocarbonée définie pour les $R_0$,$R_1$, $R_2$, $R_3$, $R_4$, $R_5$, $R_6$, $R_7$, $R_8$, $R_9$, $R_{10}$ $R_{11}$, $R_{12}$, et $R_{14}$ peuvent être remplacés par un atome d'oxygène, d'azote ou de soufre ou par un groupement $SO_2$, et/ou que la chaîne hydrocarbonée est insaturée, cela signifie que l'on peut, à titre d'exemple, faire les transformations suivantes :

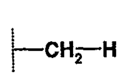 peut devenir 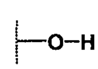

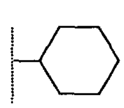 peut devenir 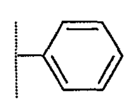

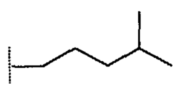 peut devenir 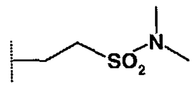

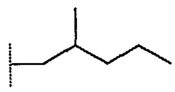 peut devenir 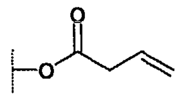

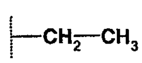 peut devenir 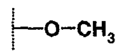

En particulier, on entend par "chaîne hydrocarbonée ramifiée", une chaîne pouvant former un ou plusieurs cycles carbonés comportant de 3 à 6 chaînons. On entend par chaîne hydrocarbonée insaturée, une chaîne pouvant comporter une ou plusieurs liaisons doubles et/ou une ou plusieurs liaisons triples, cette chaîne hydrocarbonée pouvant conduire à des groupements aromatiques.

X est un anion organique ou minéral choisi, par exemple, dans le groupe formé par un halogénure tel que chlorure, bromure, fluorure, iodure ; un hydroxyde ; un sulfate ; un hydrogénosulfate; un alkyl($C_1$-$C_6$)sulfate tel que par exemple un méthylsulfate ou un éthylsulfate ; un acétate ; un tartrate ; un oxalate ; un alkyl($C_1$-$C_6$)sulfonate tel que méthylsulfonate ; un arylsulfonate substitué ou non substitué par un radical alkyle en $C_1$-$C_4$ tel que par exemple un 4-toluylsulfonate.

De préférence, $R_1$, $R_2$, et $R_3$ représentent, identiques ou différents, un atome d'hydrogène; un radical alcoxy; hydroxy; amino; ou acétoxy ; un radical alkyle en $C_1$-$C_4$ linéaire ou ramifié substitué par un ou plusieurs radicaux choisis parmi un atome d'halogène, un radical hydroxy, alcoxy en $C_1$-$C_2$, (poly)hydroxyalcoxy, amino, (di)alkylamino en $C_1$-$C_2$, carboxyle ou sulfonique ; un groupement $-NR_{16}R_{17}$, $R_{16}$ et $R_{17}$ représentant, identiques ou différents, un atome d'hydrogène, un radical méthylsulfonyle, carboxyle, carboxamido, un radical alkyle en $C_1$-$C_4$ substitué par un ou plusieurs radicaux choisis parmi un atome d'halogène, un radical hydroxy, alcoxy en $C_1$-$C_2$, amino, (di)alkylamino en $C_1$-$C_2$, ou carboxyle;

Les carbones asymétriques desdits hétérocycles à 5, 6, 7 ou 8 chaînons de formule (II) peuvent être, indépendamment l'un de l'autre, de configuration (R) ou (S).

Plus préférentiellement, $R_1$, $R_2$, et $R_3$ représentent, identiques ou différents, un atome d'hydrogène ; un radical hydroxy, amino éventuellement mono ou di-substitué; un radical alkyle en $C_1$-$C_3$ linéaire ou ramifié, éventuellement substitué par un ou plusieurs radicaux choisis parmi les radicaux hydroxy, alcoxy en $C_1$-$C_2$, amino, (di)alkylamino en $C_1$-$C_2$, carboxyle ou sulfonique.

Encore plus particulièrement, $R_1$, $R_2$, et $R_3$ représentent, identiques ou différents, un atome d'hydrogène ; un radical méthyle; hydroxy; amino; (di)méthylamino; méthylsulfonylamino; (poly)-hydroxyalkylamino en $C_2$-$C_4$; hydroxyméthyle.

Selon l'invention et de préférence, dans la formule (II), $R_1$ est choisi dans le groupe formé par un atome d'hydrogène, un radical méthyle, un hydroxy, un amino, un méthylamino, un diméthylamino, un 2-hydroxyéthylamino, et plus particulièrement dans le groupe formé par un atome d'hydrogène, un hydroxy, un amino, un diméthylamino et plus encore désigne un atome d'hydrogène ou un radical hydroxy.

Selon l'invention et de préférence, dans la formule (II), $R_0$ est choisi dans le groupe formé par un atome d'hydrogène, un radical alkyle en $C_1$-$C_4$ linéaire ou ramifié substitué par un ou plusieurs radicaux choisis parmi les radicaux hydroxy, méthoxy, amino, (di)alkylamino, carboxyle, sulfonyle, amido, diméthylamido, et plus particulièrement $R_0$ est choisi dans le groupe formé par un atome d'hydrogène, un radical carboxyle, ou amido, ou diméthylamido ou hydroxyméthyle et plus encore désigne un atome d'hydrogène.

De préférence, $Z_1$ représente le radical $NR_{14}$ ou $CHR_2$ et plus particulièrement le radical $CHR_2$.

Dans un mode de réalisation plus préféré encore, $Z_1$ dans la formule (II) désigne $CH_2$, CHOH, $CHNH_2$ ou $CHN(CH_3)_2$ et plus particulièrement encore désigne $CH_2$.

Selon un mode de réalisation préféré, n dans la formule (II) représente les valeurs 0 ou 1 et de préférence 0.

Les radicaux $R_5$, $R_6$, $R_7$, $R_8$ $R_9$, $R_{10}$, $R_{11}$ et $R_{12}$, identiques ou différents, sont de préférence choisis dans le groupe formé par un atome d'hydrogène, un radical méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 2-hydroxyéthyloxy, 3-aminoéthyloxy, amino, méthylamino, diméthylamino, ou 2-hydroxyéthylamino.

Selon un mode de réalisation préféré, $R_5$, $R_6$, $R_7$, $R_8$, $R_9$, $R_{10}$, $R_{11}$ et $R_{12}$ , identiques ou différents, sont choisis dans le groupe formé par un atome d'hydrogène, un radical méthyle, hydroxyméthyle, 2-hydroxyéthyle, 1,2-dihydroxyéthyle, méthoxy, 2-hydroxyéthyloxy, amino, 2-hydroxyéthylamino, et plus préférentiellement encore désignent un atome d'hydrogène, un radical méthyle, méthoxy, ou amino.

Selon l'invention, $X_1$ de la formule (III) désigne de préférence le radical $CR_7$, $R_7$ étant défini comme ci-dessus et $X_2$ de la formule (III) désigne le radical $CR_8$, $R_8$ étant défini comme ci-dessus.

Selon l'invention, $R_{13}$ et $R_{15}$ , identiques ou différents, représentent de préférence un radical alkyle en $C_1$-$C_4$, éventuellement substitué par un ou plusieurs radicaux choisis dans le groupe formé les radicaux hydroxy, alcoxy en $C_1$-$C_2$, amino, (di)alkylamino en $C_1$-$C_2$, carboxyle, sulfonique, phényle, et préférentiellement méthyle, éthyle, 2-hydroxyéthyle, 1-carboxyméthyle, 2-carboxyéthyle ou 2-sulfonyléthyle. Plus préférentiellement, $R_{13}$ et $R_{15}$, identiques ou différents, représentent un alkyle en $C_1$-$C_3$ éventuellement substitué par un ou plusieurs radicaux hydroxy, méthoxy, amino, diméthylamino, carboxyle, sulfonique.

Plus particulièrement encore, $R_{13}$ et $R_{15}$ , identiques ou différents, représentent un radical méthyle, éthyle, 2-hydroxyéthyle, carboxyméthyle et encore plus particulièrement un radical méthyle.

Selon l'invention, $R_{14}$ désigne de préférence un atome d'hydrogène, un radical alkyle en $C_1$-$C_3$ linéaire ou ramifié, éventuellement substitué par un ou plusieurs radicaux choisis parmi les radicaux hydroxy, méthoxy, amino, méthylamino, diméthylamino, carboxyle ou sulfonyle.

Selon un mode de réalisation préféré, $R_{14}$ désigne un atome d'hydrogène, un radical méthyle ou un radical 2-hydroxyéthyle.

Selon un mode de réalisation préféré, $Z_2$ désigne $NR_{15}$ avec toutes les désignations prévues pour $R_{15}$ ci-dessus mentionnées.

Selon un mode de réalisation particulier, $R_0$, $R_1$, $Z_1$, $R_3$, $R_4$ et n sont choisis parmi les combinaisons suivantes :

| $R_0$ | $R_1$ | $Z_1$ | $R_3$ | $R_4$ | n |
|---|---|---|---|---|---|
| H | H | $CH_2$ | H | H | 0, 1, 2, 3 |
| H | H | N $CH_3$ | H | H | 0 ou 1 |
| H | H | N-$CH_2$-$CH_2$-OH | H | H | 0 ou 1 |
| $CH_3$ | $CH_3$ | $CH_2$ | H | H | 0 ou 1 |
| COOH | H | $CH_2$ | H | H | 0, 1, 2 |
| COOH | OH | $CH_2$ | H | H | 0 ou 1 |
| COOH | H | CH-OH | H | H | 0 |
| COOH | H | CH-COOH | H | H | 0 |
| $CH_2$-OH | OH | CH-OH | H | H | 0 |
| $CH_2$-O $CH_3$ | H | $CH_2$ | $CH_2$-O $CH_3$ | H | 0 |
| $CONH_2$ | H | $CH_2$ | H | H | 0, 1, 2 |
| $CONH_2$ | OH | $CH_2$ | H | H | 0 ou 1 |
| $CON(CH_3)_2$ | H | $CH_2$ | H | H | 0, 1, 2 |
| $CON(CH_3)_2$ | OH | $CH_2$ | H | H | 0 ou 1 |
| $CH_2OH$ | H | $CH_2$ | H | H | 0 ou 1 |
| $CH_2OH$ | OH | $CH_2$ | H | H | 0 ou 1 |
| $CH_2OH$ | H | $CH_2$ | $CH_2OH$ | H | 0 |
| H | OH | $CH_2$ | H | H | 0 ou 1 |
| H | OH | CH-OH | H | H | 0 ou 1 |
| H | $NH_2$ | $CH_2$ | H | H | 0 ou 1 |
| H | NH $CH_3$ | $CH_2$ | H | H | 0 ou 1 |
| H | $N(CH_3)_2$ | $CH_2$ | H | H | 0 ou 1 |

(suite)

| $R_0$ | $R_1$ | $Z_1$ | $R_3$ | $R_4$ | n |
|---|---|---|---|---|---|
| H | OH | $CH\text{-}NH_2$ | H | H | 0 ou 1 |
| H | OH | $CH\text{-}NH\ CH_3$ | H | H | 0 ou 1 |
| H | OH | $CH\text{-}NH\text{-}CH_2\text{-}CH\text{-}OH$ | H | H | 0 ou 1 |
| H | OH | CH-OH | OH | OH | 2 |
| $CH_3$ | H | $CH_2$ | H | H | 1 |
| H | H | CH-OH | H | H | 1 |
| $CH_3$ | H | $CH_2$ | H | $CH_3$ | 1 |
| $CH_2\text{-}NH_2$ | H | $CH_2$ | H | $CH_3$ | 1 |
| $CH_3$ | H | $CH\text{-}NH_2$ | H | H | 1 |
| H | $CH_2\text{-}OH$ | $CH_2$ | H | H | 1 |
| H | H | CH-CH-OH | H | H | 1 |
| H | $CO\text{-}NH_2$ | $CH_2$ | H | H | 1 |
| H | COOH | $CH_2$ | H | H | 1 |
| H | H | $CH_2\text{-}COOH$ | H | H | 1 |
| H | H | $CH_2$ | H | H | 1 |
| H | H | $CH\text{-}N(CH_3)_2$ | H | H | 1 |

Plus particulièrement encore, $R_0$, $R_1$, $Z_1$, $R_3$, $R_4$ et n sont choisis parmi les combinaisons suivantes :

| $R_0$ | $R_1$ | $Z_1$ | $R_3$ | $R_4$ | n |
|---|---|---|---|---|---|
| COOH | H | $CH_2$ | H | H | 0 |
| COOH | OH | $CH_2$ | H | H | 0 |
| $CON(CH_3)_2$ | H | $CH_2$ | H | H | 0 |
| $CONH_2$ | H | $CH_2$ | H | H | 0 |
| $CH_2OH$ | H | $CH_2$ | H | H | 0 |
| $CH_2OH$ | OH | $CH_2$ | H | H | 0 |
| H | OH | $CH_2$ | H | H | 0 |
| H | $NH_2$ | $CH_2$ | H | H | 0 |
| H | $NH(CH_3)_2$ | $CH_2$ | H | H | 0 |

Parmi les colorants monoazoïques monocationiques de formule (I), on peut citer notamment de préférence, les:

1,3-diméthyl-2-[4-(pyrrolidin-1-yl)-phénylazo]benzimidazol-1-ium,
1,3-diméthyl-2-[4-(3-hydroxypyrrolidin-1-yl)-phénylazo]benzimidazol-1-ium,
1,3-diméthyl-2-[4-(2-carboxypyrrolidin-1-yl)-phénylazo]benzimidazol-1-ium,
1,3-diméthyl-2-[4-(3-aminopyrrolidin-1-yl)-phénylazo]benzimidazol-1-ium,
1,3-diméthyl-2-[4-(2-carboxy-3-hydroxypyrrolidin-1-yl)-phénylazo]benzimidazol-1-ium,
1,3-diméthyl-2-[4-(2-carboxamidopyrrolidin-1-yl)-phénylazo]benzimidazol-1-ium,
1,3-diméthyl-2-[4-(2-hydroxyméthyl-pyrrolidin-1-yl)-phénylazo]benzimidazol-1-ium,
1,3-diméthyl-2-[4-(2-carboxy-4-hydroxy-pyrrolidin-1-yl)-phénylazo]benzimidazol-1-ium,
1,3-diméthyl-2-[4-(pipéridin-1-yl)-phénylazo]benzimidazol-1-ium,
1,3-diméthyl-2-[4-(3-hydroxypipéridin-1-yl)-phénylazo]benzimidazol-1-ium,

1,3-diméthyl-2-[4-(3-hydroxyméthylpipéridin-1-yl)-phénylazo]benzimidazol-1-ium,
1,3-diméthyl-2-[4-(3-carboxypipéridin-1-yl)-phénylazo]benzimidazol-1-ium,
1,3-diméthyl-2-[4-(2-carboxypipéridin-1-yl)-phénylazo]benzimidazol-1-ium,
1,3-diméthyl-2-[4-(pipérazin-1-yl)-phénylazo]benzimidazol-1-ium,
1,3-diméthyl-2-[4-(homopipérazin-1-yl)-phénylazo]benzimidazol-1-ium,

5-amino-1,3-diméthyl-2-[4-(pyrrolidin-1-yl)-phénylazo]benzimidazol-1-ium,
5-amino-1,3-diméthyl-2-[4-(3-hydroxypyrrolidin-1-yl)-phénylazo]benzimidazol-1-ium,
5-amino-1,3-diméthyl-2-[4-(2-carboxypyrrolidin-1-yl)-phénylazo]benzimidazol-1-ium,
5-amino-1,3-diméthyl-2-[4-(3-aminopyrrolidin-1-yl)-phénylazo]benzimidazol-1-ium,
5-amino-1,3-diméthyl-2-[4-(2-carboxy-3-hydroxypyrrolidin-1-yl)-phénylazo]benzimidazol-1-ium,
5-amino-1,3-diméthyl-2-[4-(2-carboxamidopyrrolidin-1-yl)-phénylazo]benzimidazol-1-ium,
5-amino-1,3-diméthyl-2-[4-(2-hydroxyméthyl-pyrrolidin-1-yl)-phénylazo]benzimidazol-1-ium,
5-amino-1,3-diméthyl-2-[4-(2-carboxy-4-hydroxy-pyrrolidin-1-yl)-phénylazo]benzimidazol-1-ium,
5-amino-1,3-diméthyl-2-[4-(pipéridin-1-yl)-phénylazo]benzimidazol-1-ium,
5-amino-1,3-diméthyl-2-[4-(3-hydroxypipéridin-1-yl)-phénylazo]benzimidazol-1-ium,
5-amino-1,3-diméthyl-2-[4-(3-hydroxyméthylpipéridin-1-yl)-phénylazo]benzimidazol-1-ium,
5-amino-1,3-diméthyl-2-[4-(3-carboxypipéridin-1-yl)-phénylazo]benzimidazol-1-ium,
5-amino-1,3-diméthyl-2-[4-(2-carboxypipéridin-1-yl)-phénylazo]benzimidazol-1-ium,
5-amino-1,3-diméthyl-2-[4-(pipérazin-1-yl)-phénylazo]benzimidazol-1-ium,
5-amino-1,3-diméthyl-2-[4-(homopipérazin-1-yl)-phénylazo]benzimidazol-1-ium,

5-diméthylamino-1,3-diméthyl-2-[4-(pyrrolidin-1-yl)-phénylazo]benzimidazol-1-ium,
5-diméthylamino-1,3-diméthyl-2-[4-(3-hydroxypyrrolidin-1-yl)-phénylazo]benzimidazol-1-ium,
5-diméthylamino-1,3-diméthyl-2-[4-(2-carboxypyrrolidin-1-yl)-phénylazo]benzimidazol-1-ium,
5-diméthylamino-1,3-diméthyl-2-[4-(3-aminopyrrolidin-1-yl)-phénylazo]benzimidazol-1-ium,
5-diméthylamino-1,3-diméthyl-2-[4-(2-carboxy-3-hydroxypyrrolidin-1-yl)-phénylazo]benzimidazol-1-ium,
5-diméthylamino-1,3-diméthyl-2-[4-(2-carboxamidopyrrolidin-1-yl)-phénylazo]benzimidazol-1-ium,
5-diméthylamino-1,3-diméthyl-2-[4-(2-hydroxyméthyl-pyrrolidin-1-yl)-phénylazo]benzimidazol-1-ium,
5-diméthylamino-1,3-diméthyl-2-[4-(2-carboxy-4-hydroxy-pyrrolidin-1-yl)-phénylazo]benzimidazol-1-ium,
5-diméthylamino-1,3-diméthyl-2-[4-(pipéridin-1-yl)-phénylazo]benzimidazol-1-ium,
5-diméthylamino-1,3-diméthyl-2-[4-(3-hydroxypipéridin-1-yl)-phénylazo]benzimidazol-1-ium,
5-diméthylamino-1,3-diméthyl-2-[4-(3-hydroxyméthylpipéridin-1-yl)-phénylazo]benzimidazol-1-ium,
5-diméthylamino-1,3-diméthyl-2-[4-(3-carboxypipéridin-1-yl)-phénylazo]benzimidazol-1-ium,
5-diméthylamino-1,3-diméthyl-2-[4-(2-carboxypipéridin-1-yl)-phénylazo]benzimidazol-1-ium,
5-diméthylamino-1,3-diméthyl-2-[4-(pipérazin-1-yl)-phénylazo]benzimidazol-1-ium,
5-diméthylamino-1,3-diméthyl-2-[4-(homopipérazin-1-yl)-phénylazo]benzimidazol-1-ium,

La concentration en colorant monocationique monoazoïque de formule (I) peut varier d'environ 0,001 à 5% en poids par rapport au poids total de la composition tinctoriale, et de préférence d'environ 0,05 à 2%.

La composition tinctoriale conforme à l'invention peut en outre contenir des colorants directs différents de ceux de formule (I), ces colorants pouvant notamment être choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

Parmi les colorants directs benzéniques, on peut citer notamment les composés suivants:

- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène

- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoiques cationiques décrits dans les demandes de brevets WO 95/15144, WO-95/01772 et EP-714954 dont le contenu fait partie intégrante de l'invention.
Parmi ces composés on peut tout particulièrement citer les colorants suivants:

- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3$^{e}$ édition :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.
Parmi les colorants directs quinoniques on peut citer les colorants suivants :

- Disperse Red 15
- Solvent Violet 13
- Acid Violet 43
- Disperse Violet 1
- Disperse Violet 4

- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99

ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :

- Basic Blue 17
- Basic Red 2.

Parmi les colorants triarylméthaniques, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques, on peut citer les composés suivants :

- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné. Le ou les colorants directs additionnels représentent de préférence de 0,001 à 20% en poids environ du poids total de la composition et encore plus préférentiellement de 0,005 à 10% en poids environ.

La composition de l'invention peut en outre comprendre un agent oxydant. Cet agent oxydant peut être n'importe quel agent oxydant utilisé de façon classique pour la décoloration des fibres kératiniques humaines. L'agent oxydant est choisi de préférence parmi le peroxyde d'hydrogène, le peroxyde d'urée, les bromates de métaux alcalins, les persels tels que les perborates et les persulfates, les peracides et les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Lorsque la composition selon l'invention est destinée à une teinture d'oxydation classique, elle comprend en outre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétraaminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino

1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir en outre un ou plusieurs coupleurs conventionnellement utilisés pour la teinture d'oxydation classique de fibres kératiniques humaines. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général présents en quantité comprise entre 0,001 et 10 % en poids environ du poids total de la composition tinctoriale et plus préférentiellement de 0,005 à 6 %. La ou les bases d'oxydation sont présentes en quantité de préférence comprise entre 0,001 à 10 % en poids environ du poids total de la composition tinctoriale, et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Le milieu cosmétiquement acceptable appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante :

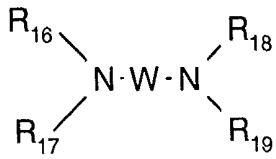

(V)

dans laquelle W est un reste propylène éventuellement substitué par un groupement hydroxyle ou un radical alkyle en $C_1$-$C_4$ ; $R_{16}$, $R_{17}$, $R_{18}$ et $R_{19}$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques humaines, et notamment des cheveux.

L'invention a aussi pour objet un procédé de teinture directe qui comprend l'application d'une composition tinctoriale contenant un colorant de formule (I) telle que définie précédemment sur les fibres kératiniques humaines. Après un temps de pause, les fibres sont rincées laissant apparaître des fibres colorées.

L'application sur les fibres de la composition tinctoriale contenant le colorant de formule (I) peut être mise en oeuvre en présence d'agent oxydant ce qui provoque la décoloration de la fibre (teinture directe éclaircissante). Cet agent oxydant peut être ajouté à la composition contenant le colorant monoazoïque monocationique au moment de l'emploi ou directement sur la fibre.

L'invention a aussi pour objet un procédé de teinture d'oxydation qui comprend l'application sur les fibres kératiniques humaines d'une composition tinctoriale qui comprend un colorant de formule (I), au moins une base d'oxydation et optionnellement au moins un coupleur, en présence d'un agent oxydant.

La base d'oxydation, le coupleur et l'agent oxydant sont tels que définis précédemment.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.

Dans le cas de la teinture d'oxydation ou de la teinture directe, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres. Après un temps de pause de 3 à 50 minutes environ, de préférence 5 à 30 minutes environ, les fibres sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques humaines et tels que définis précédemment.

La composition qui est finalement appliquée sur les fibres peut se présenter sous des formes diverses, telles que sous forme de liquides, de crèmes, de gels ou sous toute autre forme appropriée pour réaliser une teinture des fibres kératiniques humaines, et notamment des cheveux.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet les nouveaux composés monoazoïques monocationiques de formule (I) telle que définie précédemment et pour laquelle dans la formule (II), n désigne les valeurs 0, 2 ou 3.

L'invention a également pour objet l'utilisation des colorants monoazoïques monocationiques de formule (I), à titre de colorants directs, dans une composition de teinture des fibres kératiniques humaines, et plus particulièrement des cheveux.

Les composés de l'invention peuvent être obtenus classiquement à partir d'un sel de diazonium de 2-aminobenzimidazole. La préparation du diazonium est connue et décrite dans la littérature, et elle est réalisée en milieu acide chlorhydrique dilué.

Le sel de diazonium est ensuite condensé sur un composé de la famille des N,N-dialkyles cycliques d'anilines en présence d'une base organique.

A la suite de cette condensation, le milieu réactionnel est ramené à pH basique et l'azoïque ainsi formé est

isolé.

L'azoïque ainsi obtenu est quaternisé dans un solvant à une température comprise entre 40°C et 140°C à l'aide d'un agent alkylant.

Ainsi, par exemple, les dérivés de 2-aminobenzimidazoles réagissent avec le nitrite de sodium en milieu acide pour conduire à l'intermédiaire réactif diazonium, qui lui-même est mis en réaction avec un dérivé d'aniline cyclique. Les préparations sont connues et bien décrites dans les brevets US-5 208 325 et US- 5 436 323. Le protocole est également appliqué aux dérivés de 2-aminobenzoxazoles et 2 aminobenzothiazoles.

Les composés azoïques correspondants sont ensuite quaternisés par les halogénures $R_{13}$-X suivant le schéma réactionnel suivant :

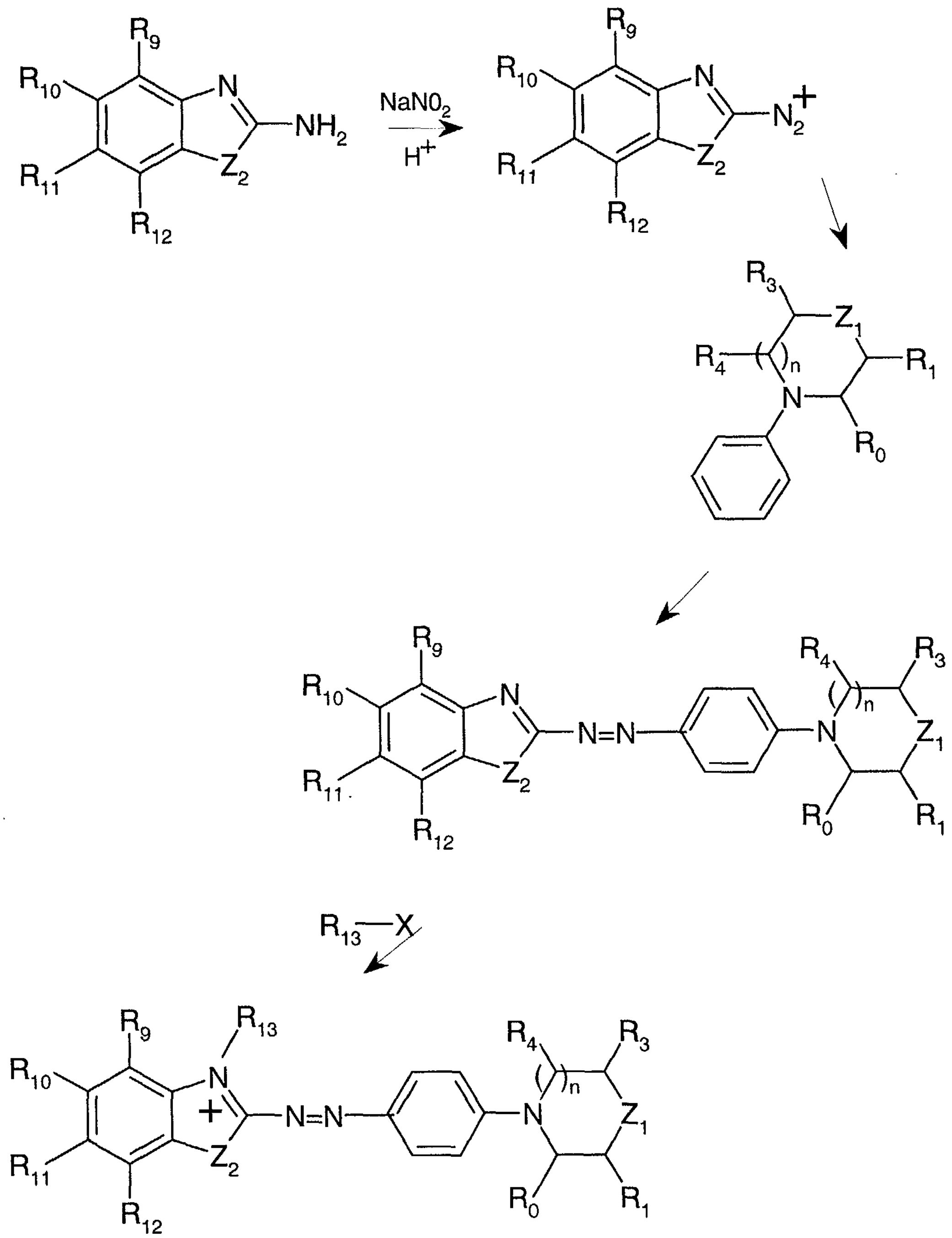


En appliquant le même procédé de synthèse aux dérivés de pyridines et de pyridazines substitués par une amine cyclique de 5 à 8 chaînons, les colorants monoazoïques monocationiques de formule (I) correspondants sont

obtenus.

Les exemples qui suivent servent à illustrer l'invention sans toutefois présenter un caractère limitatif.

**EXEMPLE DE SYNTHESE**

On a préparé le composé conforme à l'invention de formule suivante:

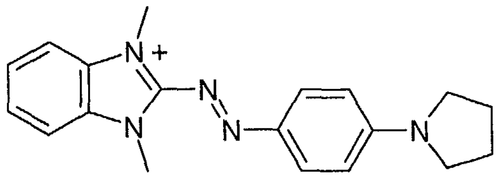

$CH_3SO_4^-$

Première étape

Synthèse du (1H-Benzoimidazol-2-yl)-(4-pyrrolidin-1-yl-phényl)-diazène de formule:

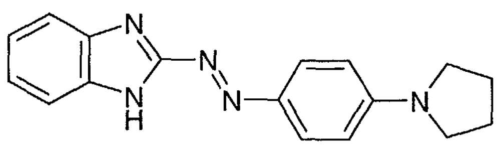

Dans un ballon tout équipé, on a solubilisé 5.42g ($4.10^{-2}$mole), de 2-aminobenzimidazole dans 400 ml d'acide orthophosphorique.

Après refroidissement à 0° C, on a coulé en 30 minutes une solution de nitrite de sodium ( 3.92g dans 20ml d'eau), puis on a agité pendant 15minutes.

On a ensuite ajouté en 30 minutes et sous agitation, 1,5 kg de glace.

Puis on a ajouté une solution éthanolique de 1-phénylpyrrolidine (5.4g dans 20ml d'éthanol) au goutte à goutte pendant 30 minutes.

A 0°C, le pH du milieu a été ramené à 7 à l'aide d'une solution d'ammoniaque et le produit rouge brique formé a été essoré sur fritté n°4 puis lavé avec 10 fois 100ml d'eau.

Après séchage, on a récupéré 2 grammes de produit rouge brique.

Seconde étape

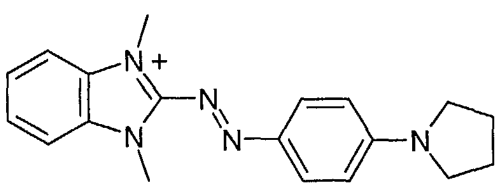

$CH_3SO_4^-$

Dans un ballon tout équipé, on a chargé 1.51g (5.2 $10^{-3}$mole), de (1H-benzoimidazol-2-yl)-(4-pyrrolidin-1-yl-phényl)-diazène obtenu comme décrit si dessus, 10 ml de dichloroéthane anhydre, 1.4g ($10^{-3}$mole) d'acétate de sodium et 1.08ml de diméthylsufate.

Le mélange a été porté 1h30 heure à 50°C sous agitation. On a essoré le solide que l'on a lavé au dichloroéthane, puis on l'a séché. On a obtenu 2.08 g de poudre violette foncée présentant les caractéristiques suivantes en absorption UV :

UV (éthanol) $\lambda_{max}$ = 576 nm

$\varepsilon_{max}$ = 30.000

RMN 1H : (400MHz-DMSO) ppm : conforme

**EXEMPLE D'APPLICATION**

Une composition colorante comprenant 1,25 gramme de colorant préparé à l'exemple précédent, 13 grammes d'une solution aqueuse d'ammoniaque titrant 40% d'ammoniac en poids et de l'eau déminéralisée q.s.p. 100 grammes a été mélangée au moment de l'emploi avec une composition d'eau oxygénée à 20 volumes à raison de 1 partie de composition colorante pour 1,5 partie de composition oxydante. Le mélange obtenu a été appliqué sur des mèches de cheveux naturels et permanentés à 90% de blancs (1 gramme de cheveux pour 10 grammes de mélange).

La composition a été laissé posée 35 minutes à température ambiante.

Les mèches ont ensuite été rincées à l'eau, shampooinées puis séchées.

Elles ont été teintes dans un nuance irisé violine à violine bien résistante à la lumière.

**Revendications**

**1.** Composition pour la teinture des fibres kératiniques humaines, et plus particulièrement des cheveux, comprenant dans un milieu cosmétiquement acceptable, au moins un colorant monoazoïque monocationique de formule (I) suivante :

$$W_1—W_2—N{=}N—W_3 \qquad (I)$$

dans laquelle

$W_1$ représente un hétérocycle à 5, 6,7 ou 8 chaînons de formule (II) suivante

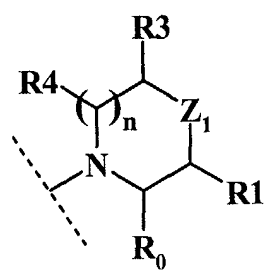

formule (II)

$W_2$ représente un groupe divalent aromatique carboné, pyridinique ou pyridazinique de formule (III) suivante

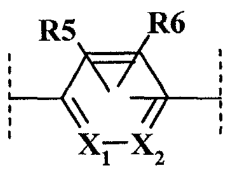

formule (III)

$W_3$ représente un radical hétéroaromatique cationique représenté par la formule (IV) suivante :

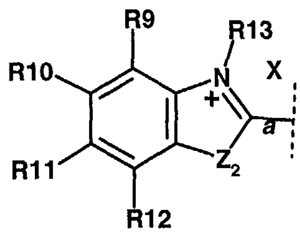

(IV)

Formules (II), (III), (IV) dans lesquelles :

- n = 0, 1, 2 ou 3, étant entendu que lorsque n est supérieur ou égal à 2, alors les radicaux $R_4$ peuvent être identiques ou différents,
- $X_1$ représente un atome d'azote ou un radical $CR_7$,
- $X_2$ représente un atome d'azote ou un radical $CR_8$,
- $Z_1$ représente un radical $CHR_2$ , un atome d'oxygène, de soufre ou un radical $NR_{14}$,
- $Z_2$ représente un atome d'oxygène, de soufre ou un radical $NR_{15}$
- $R_{0,}$ $R_1$, $R_2$, $R_3$, $R_4$, $R_5$, $R_6$, $R_7$, $R_8$, $R_9$, $R_{10}$, $R_{11}$ et $R_{12}$ représentent, identiques ou différents, un atome d'hydrogène, une chaîne hydrocarbonée en $C_1$-$C_{10}$ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement $SO_2$, et dont les atomes de carbone peuvent être, indépendamment les uns des autres, substitués par un ou plusieurs atomes d'halogène ; $R_{0,}$ $R_1$, $R_2$, $R_3$, $R_4$, $R_5$, $R_6$, $R_7$, $R_8$, $R_9$, $R_{11}$ et $R_{12}$ ne comportant pas de liaison peroxyde, ni de radicaux diazo ou nitroso,
- $R_{14}$ représente un atome d'hydrogène, une chaîne hydrocarbonée en $C_1$-$C_{10}$ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement $SO_2$, et dont les atomes de carbone peuvent être, indépendamment les uns des autres, substitués par un ou plusieurs atomes d'halogène, $R_{14}$ ne comportant pas de liaison peroxyde, ni de radicaux diazo ou nitroso ; étant entendu que lesdits atomes d'oxygène, d'azote et de soufre ne sont pas reliés directement à l'atome d'azote porteur du radical $R_{14}$.
- $R_5$ avec $R_6$ peuvent former un cycle aromatique carboné, tel qu'un phényle,
- $R_{13}$ et $R_{15}$ représentent, identiques ou différents, un radical alkyle en $C_1$-$C_8$, éventuellement substitué par un ou plusieurs radicaux choisis dans le groupe consitué par un hydroxy, un alcoxy en $C_1$-$C_2$, un radical (poly) hydroxyalcoxy en $C_2$-$C_4$, un amino, un (di)-alkylamino en $C_1$-$C_2$, un carboxy, un sulfonique, un phényl éventuellement substitué ;
- la liaison **a** du cycle cationique de la formule (IV) est reliée au groupement azoïque de la formule (I);
- X est un anion organique ou minéral.

**2.** Composition selon la revendication 1, **caractérisée par le fait que** dans la formule (I), $R_1$, $R_{2,}$ et $R_3$ identiques ou différents, représentent, un atome d'hydrogène; un radical alcoxy; hydroxy; amino; ou acétoxy ; un radical alkyle en $C_1$-$C_4$ linéaire ou ramifié substitué par un ou plusieurs radicaux choisis parmi un atome d'halogène, un radical

hydroxy, alcoxy en $C_1$-$C_2$, (poly)hydroxyalcoxy, amino, (di)alkylamino en $C_1$-$C_2$, carboxyle ou sulfonique ; un groupement -$NR_{16}R_{17}$, $R_{16}$ et $R_{17}$ représentant, identiques ou différents, un atome d'hydrogène, un radical méthylsulfonyle, carboxyle, carboxamido, un radical alkyle en $C_1$-$C_4$ substitué par un ou plusieurs radicaux choisis parmi un atome d'halogène, un radical hydroxy, alcoxy en $C_1$-$C_2$, amino, (di)alkylamino en $C_1$-$C_2$, ou carboxyle.

**3.** Composition selon la revendication 2 dans laquelle $R_1$, $R_2$, et $R_3$ représentent, identiques ou différents, un atome d'hydrogène ; un radical hydroxy, amino éventuellement mono ou di-substitué; un radical alkyle en $C_1$-$C_3$ linéaire ou ramifié, éventuellement substitué par un ou plusieurs radicaux choisis parmi les radicaux hydroxy, alcoxy en $C_1$-$C_2$, amino, (di)alkylamino en $C_1$-$C_2$, carboxyle ou sulfonique.

**4.** Composition selon la revendication 3 dans laquelle $R_1$, $R_2$, et $R_3$ représentent, identiques ou différents, un atome d'hydrogène ; un radical méthyle; hydroxy; amino; (di)méthylamino; méthylsulfonylamino; (poly)-hydroxyalkylamino en $C_2$-$C_4$ ; hydroxyméthyle.

**5.** Composition selon l'une quelconque des revendications précédentes dans laquelle, $R_1$ dans la formule (II), est choisi dans le groupe formé par un atome d'hydrogène, un radical méthyle, un hydroxy, un amino, un méthylamino, un diméthylamino, un 2-hydroxyéthylamino, et plus particulièrement dans le groupe formé par un atome d'hydrogène, un hydroxy, un amino, un diméthylamino et plus encore désigne un atome d'hydrogène ou un radical hydroxy.

**6.** Composition selon l'une quelconque des revendications précédentes dans laquelle, $R_0$ dans la formule (II), est choisi dans le groupe formé par un atome d'hydrogène, un radical alkyle en $C_1$-$C_4$ linéaire ou ramifié substitué par un ou plusieurs radicaux choisis parmi les radicaux hydroxy, méthoxy, amino, (di)alkylamino, carboxyle, sulfonyle, amido, diméthylamido, et plus particulièrement $R_0$ est choisi dans le groupe formé par un atome d'hydrogène, un radical carboxyle, ou amido, ou diméthylamido ou hydroxyméthyle et plus encore désigne un atome d'hydrogène.

**7.** Composition selon l'une quelconque des revendications précédentes dans laquelle $Z_1$ dans la formule (II), représente le radical $NR_{14}$ ou $CHR_2$ et de préférence $CHR_2$.

**8.** Composition selon la revendication 7 dans laquelle $Z_1$ désigne $CH_2$, CHOH, $CHNH_2$ ou $CHN(CH_3)_2$ et plus particulièrement $CH_2$.

**9.** Composition selon l'une quelconque des revendications précédentes dans laquelle n dans la formule (II), représente les valeurs 0 ou 1 et de préférence 0.

**10.** Composition selon l'une quelconque des revendications précédentes dans laquelle $R_5$, $R_6$, $R_7$, $R_8$, $R_9$, $R_{10}$, $R_{11}$ et $R_{12}$ , identiques ou différents, sont choisis dans le groupe formé par un atome d'hydrogène, un radical méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, méthoxy, éthoxy, 2-hydroxyéthyloxy, 3-aminoéthyloxy, amino, méthylamino, diméthylamino, ou 2-hydroxyéthylamino.

**11.** Composition selon la revendication 10 dans laquelle $R_5$, $R_6$, $R_7$, $R_8$, $R_9$, $R_{10}$, $R_{11}$ et $R_{12}$ , identiques ou différents, sont choisis dans le groupe formé par un atome d'hydrogène, un radical méthyle, hydroxyméthyle, 2-hydroxyéthyle, 1,2-dihydroxyéthyle, méthoxy, 2-hydroxyéthyloxy, amino, 2-hydroxyéthylamino, et plus préférentiellement dans le groupe formé par un atome d'hydrogène, un radical méthyle, méthoxy, ou amino.

**12.** Composition selon l'une quelconque des revendications précédentes dans laquelle $X_1$ dans la formule (III) désigne le radical $CR_7$, $R_7$ étant défini selon l'une quelconque des revendications 10 ou 11.

**13.** Composition selon l'une quelconque des revendications précédentes dans laquelle $X_2$ dans la formule (III) désigne le radical $CR_8$, $R_8$ étant défini selon l'une quelconque des revendications 10 ou 11.

**14.** Composition selon l'une quelconque des revendications précédentes dans laquelle $R_{13}$ et $R_{15}$ représentent, identiques ou différents, un radical alkyle en $C_1$-$C_4$, éventuellement substitué par un ou plusieurs radicaux choisis dans le groupe formé les radicaux hydroxy, alcoxy en $C_1$-$C_2$, amino, (di)alkylamino en $C_1$-$C_2$, carboxyle, sulfonique, phényle, et préférentiellement méthyle, éthyle, 2-hydroxyéthyle, 1-carboxyméthyle, 2-carboxyéthyle ou 2-sulfonyléthyle.

**15.** Composition selon la revendication 14 pour laquelle, $R_{13}$ et $R_{15}$ identiques ou différents, représentent un alkyle en $C_1$-$C_3$ éventuellement substitué par un ou plusieurs radicaux hydroxy, méthoxy, amino, diméthylamino, carboxyle, sulfonique.

**16.** Composition selon la revendication 15 pour laquelle $R_{13}$ et $R_{15}$ , identiques ou différents, représentent un radical méthyle, éthyle, 2-hydroxyéthyle, carboxyméthyle et encore plus particulièrement un radical méthyle.

**17.** Composition selon la revendication 7 dans laquelle lorsque $Z_1$ désigne $NR_{14}$, $R_{14}$ désigne un atome d'hydrogène, un radical alkyle en $C_1$-$C_3$ linéaire ou ramifié, éventuellement substitué par un ou plusieurs radicaux choisis parmi les radicaux hydroxy, méthoxy, amino, méthylamino, diméthylamino, carboxyle ou sulfonyle.

**18.** Composition selon la revendication 17 pour laquelle $R_{14}$ désigne un atome d'hydrogène, un radical méthyle ou un radical 2-hydroxyéthyle.

**19.** Composition selon l'une quelconque des revendications précédentes dans laquelle $Z_2$ dans la formule (IV) désigne $NR_{15}$.

**20.** Composition selon l'une quelconque des revendications précédentes, **caractérisée par le fait que** le colorant monoazoïque monocationique de formule (I) est choisi dans le groupe formé par les:

1,3-diméthyl-2-[4-(pyrrolidin-1-yl)-phénylazo]benzimidazol-1-ium,
1,3-diméthyl-2-[4-(3-hydroxypyrrolidin-1-yl)-phénylazo]benzimidazol-1-ium,
1,3-diméthyl-2-[4-(2-carboxypyrrolidin-1-yl)-phénylazo]benzimidazol-1-ium,
1,3-diméthyl-2-[4-(3-aminopyrrolidin-1-yl)-phénylazo]benzimidazol-1-ium,
1,3-diméthyl-2-[4-(2-carboxy-3-hydroxypyrrolidin-1-yl)-phénylazo]benzimidazol-1-ium,
1,3-diméthyl-2-[4-(2-carboxamidopyrrolidin-1-yl)-phénylazo]benzimidazol-1-ium,
1,3-diméthyl-2-[4-(2-hydroxyméthyl-pyrrolidin-1-yl)-phénylazo]benzimidazol-1-ium,
1,3-diméthyl-2-[4-(2-carboxy-4-hydroxy-pyrrolidin-1-yl)-phénylazo]benzimidazol-1-ium,
1,3-diméthyl-2-[4-(pipéridin-1-yl)-phénylazo]benzimidazol-1-ium,
1,3-diméthyl-2-[4-(3-hydroxypipéridin-1-yl)-phénylazo]benzimidazol-1-ium,
1,3-diméthyl-2-[4-(3-hydroxyméthylpipéridin-1-yl)-phénylazo]benzimidazol-1-ium,
1,3-diméthyl-2-[4-(3-carboxypipéridin-1-yl)-phénylazo]benzimidazol-1-ium,
1,3-diméthyl-2-[4-(2-carboxypipéridin-1-yl)-phénylazo]benzimidazol-1-ium,
1,3-diméthyl-2-[4-(pipérazin-1-yl)-phénylazo]benzimidazol-1-ium,
1,3-diméthyl-2-[4-(homopipérazin-1-yl)-phénylazo]benzimidazol-1-ium,

**21.** Composition selon l'une quelconque des revendications 1 à 19, **caractérisée par le fait que** le colorant monoazoïque monocationique de formule (I) est choisi dans le groupe formé par les:

5-amino-1,3-diméthyl-2-[4-(pyrrolidin-1-yl)-phénylazo]benzimidazol-1-ium,
5-amino-1,3-diméthyl-2-[4-(3-hydroxypyrrolidin-1-yl)-phénylazo]benzimidazol-1-ium,
5-amino-1,3-diméthyl-2-[4-(2-carboxypyrrolidin-1-yl)-phénylazo]benzimidazol-1-ium,
5-amino-1,3-diméthyl-2-[4-(3-aminopyrrolidin-1-yl)-phénylazo]benzimidazol-1-ium,
5-amino-1,3-diméthyl-2-[4-(2-carboxy-3-hydroxypyrrolidin-1-yl)-phénylazo]benzimidazol-1-ium,
5-amino-1,3-diméthyl-2-[4-(2-carboxamidopyrrolidin-1-yl)-phénylazo]benzimidazol-1-ium,
5-amino-1,3-diméthyl-2-[4-(2-hydroxyméthyl-pyrrolidin-1-yl)-phénylazo]benzimidazol-1-ium,
5-amino-1,3-diméthyl-2-[4-(2-carboxy-4-hydroxy-pyrrolidin-1-yl)-phénylazo]benzimidazol-1-ium,
5-amino-1,3-diméthyl-2-[4-(pipéridin-1-yl)-phénylazo]benzimidazol-1-ium,
5-amino-1,3-diméthyl-2-[4-(3-hydroxypipéridin-1-yl)-phénylazo]benzimidazol-1-ium,
5-amino-1,3-diméthyl-2-[4-(3-hydroxyméthylpipéridin-1-yl)-phénylazo]benzimidazol-1-ium,
5-amino-1,3-diméthyl-2-[4-(3-carboxypipéridin-1-yl)-phénylazo]benzimidazol-1-ium,
5-amino-1,3-diméthyl-2-[4-(2-carboxypipéridin-1-yl)-phénylazo]benzimidazol-1-ium,
5-amino-1,3-diméthyl-2-[4-(pipérazin-1-yl)-phénylazo]benzimidazol-1-ium,
5-amino-1,3-diméthyl-2-[4-(homopipérazin-1-yl)-phénylazo]benzimidazol-1-ium,

**22.** Composition selon l'une quelconque des revendications 1 à 19, **caractérisée par le fait que** le colorant monoazoïque monocationique de formule (I) est choisi dans le groupe formé par les:

5-diméthylamino-1,3-diméthyl-2-[4-(pyrrolidin-1-yl)-phénylazo]benzimidazol-1-ium,
5-diméthylamino-1,3-diméthyl-2-[4-(3-hydroxypyrrolidin-1-yl)-phénylazo]benzimidazol-1-ium,
5-diméthylamino-1,3-diméthyl-2-[4-(2-carboxypyrrolidin-1-yl)-phénylazo]benzimidazol-1-ium,
5-diméthylamino-1,3-diméthyl-2-[4-(3-aminopyrrolidin-1-yl)-phénylazo]benzimidazol-1-ium,
5-diméthylamino-1,3-diméthyl-2-[4-(2-carboxy-3-hydroxypyrrolidin-1-yl)-phénylazo]benzimidazol-1-ium,
5-diméthylamino-1,3-diméthyl-2-[4-(2-carboxamidopyrrolidin-1-yl)-phénylazo]benzimidazol-1-ium,
5-diméthylamino-1,3-diméthyl-2-[4-(2-hydroxyméthyl-pyrrolidin-1-yl)-phénylazo]benzimidazol-1-ium,
5-diméthylamino-1,3-diméthyl-2-[4-(2-carboxy-4-hydroxy-pyrrolidin-1-yl)-phénylazo]benzimidazol-1-ium,
5-diméthylamino-1,3-diméthyl-2-[4-(pipéridin-1-yl)-phénylazo]benzimidazol-1-ium,
5-diméthylamino-1,3-diméthyl-2-[4-(3-hydroxypipéridin-1-yl)-phénylazo]benzimidazol-1-ium,
5-diméthylamino-1,3-diméthyl-2-[4-(3-hydroxyméthylpipéridin-1-yl)-phénylazo]benzimidazol-1-ium,
5-diméthylamino-1,3-diméthyl-2-[4-(3-carboxypipéridin-1-yl)-phénylazo]benzimidazol-1-ium,
5-diméthylamino-1,3-diméthyl-2-[4-(2-carboxypipéridin-1-yl)-phénylazo]benzimidazol-1-ium,
5-diméthylamino-1,3-diméthyl-2-[4-(pipérazin-1-yl)-phénylazo]benzimidazol-1-ium,
5-diméthylamino-1,3-diméthyl-2-[4-(homopipérazin-1-yl)-phénylazo]benzimidazol-1-ium,

**23.** Composition selon l'une quelconque des revendications précédentes dans laquelle X désigne un halogénure, un hydroxyde, un sulfate, un hydrogénosulfate, un alkyl($C_1$-$C_6$)sulfate, un acétate, un tartrate, un oxalate, un alkyl ($C_1$-$C_6$)sulfonate, un arylsulfonate substitué ou non substitué par un radical alkyle en $C_1$-$C_4$.

**24.** Composition selon l'une quelconque des revendications précédentes, **caractérisée par le fait que** le colorant monoazoïque monocationique de formule (I) est présent à une concentration variant de 0,001 à 5% en poids par rapport au poids total de la composition tinctoriale, et de préférence de 0,05 à 2%.

**25.** Composition selon l'une quelconque des revendications 1 à 24 comprenant en outre au moins un colorant direct différent de ceux de formule (I), choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

**26.** Composition selon l'une quelconque des revendications précédentes comprenant en outre un agent oxydant, de préférence le peroxyde d'hydrogène.

**27.** Composition selon l'une quelconque des revendications 1 à 26 comprenant de plus une base d'oxydation.

**28.** Composition selon la revendication 27 dans laquelle la base d'oxydation est choisie parmi les paraphénylènediamines, les bis-phénylalkylènediamines, les para-aminophénols, les ortho-aminophénols, les bases hétérocycliques, et leurs sels d'addition avec un acide.

**29.** Composition selon l'une quelconque des revendications 26 à 28 comprenant en outre au moins un coupleur.

**30.** Composition selon la revendication 29 dans laquelle le coupleur est choisi parmi les métaphénylènediamines, les méta-aminophénols, les métadiphénols, les coupleurs naphtaléniques et les coupleurs hétérocycliques et leur sel d'addition avec un acide.

**31.** Procédé de teinture directe des fibres kératiniques humaines et plus particulièrement des cheveux, **caractérisé en ce qu'**on applique sur les fibres au moins une composition tinctoriale telle que définie à l'une quelconque des revendications 1 à 25.

**32.** Procédé selon la revendication 31 dans lequel la composition tinctoriale contient un agent oxydant.

**33.** Procédé selon la revendication 32 dans lequel l'agent oxydant est mélangé au moment de l'emploi à la composition tinctoriale.

**34.** Procédé selon la revendication 32 dans lequel l'agent oxydant est appliqué sur les fibres sous forme de composition oxydante simultanément ou séquentiellement à la composition tinctoriale.

**35.** Procédé de teinture d'oxydation des fibres kératiniques humaines et plus particulièrement des cheveux, **caractérisé en ce qu'**on applique sur les fibres au moins une composition tinctoriale telle que définie à l'une quelconque des revendications 1 à 26, comprenant de plus au moins une base d'oxydation et optionnellement au moins un coupleur, en présence d'un agent oxydant.

**36.** Procédé selon la revendication 35 dans lequel l'agent oxydant est mélangé au moment de l'emploi à la composition tinctoriale.

**37.** Procédé selon la revendication 35 dans lequel l'agent oxydant est appliqué sur les fibres sous forme de composition oxydante simultanément ou séquentiellement à la composition tinctoriale.

**38.** Dispositif à plusieurs compartiments ou "kit" de teinture à plusieurs compartiments pour la teinture des fibres kératiniques humaines, dans lequel un premier compartiment contient une composition telle que définie à l'une quelconque des revendications 1 à 25 et 27 à 30 et un deuxième compartiment contient une composition oxydante.

**39.** Composés monoazoïques monocationiques de formule (I) telle que définie à l'une quelconque des revendications 1 à 23 et pour laquelle, n dans la formule (II), désigne les valeurs 0, 2 ou 3.

**40.** Utilisation des composés monoazoïques monocationiques de formule (I) telle que définie selon l'une quelconque des revendications 1 à 23, à titre de colorants directs, dans une, ou pour la préparation d'une composition de teinture des fibres kératiniques humaines et plus particulièrement des cheveux.